# EUROPÄISCHE PATENTSCHRIFT

(11) **EP 0 281 736 B1**
(45) Veröffentlichungstag und Bekanntmachung des Hinweises auf die Patenterteilung: **16.06.1993**
(21) Anmeldenummer: 88100826.2
(22) Anmeldetag: 21.01.1988
(51) Int. Cl.: A61B 19/00

(54) **Gerät zur Lagerung und Behandlung operativ gewonnener Venenstücke**
Device for storing and treating operatively gained fragments of vein
Dispositif pour le stockage et le traitement de tronçons de veine extraits

(30) Priorität: 05.03.1987 CH 825/87
(43) Veröffentlichungstag der Anmeldung: 14.09.1988
(73) Patentinhaber: GEBRÜDER SULZER AKTIENGESELLSCHAFT, CH-8401 Winterthur (CH)
(72) Erfinder: Müller, Werner, Dr., CH-8542 Wiesendangen (CH); Hensel, Marie-Claude, CH-8614 Bertschikon (CH); Huber, August, CH-8352 Räterschen (CH)

(56) Entgegenhaltungen:
- US-A- 3 916 874
- US-A- 4 232 659

## Beschreibung

Die Erfindung betrifft ein Gerät zur Lagerung und/oder Behandlung operativ gewonnener Venenstücke, insbesondere zum Transport und/oder zur Gewinnung von Endothelzellen, die enzymatisch aus dem Venenstück herausgelöst und zur Vermehrung einer geeigneten Zellwachstumskultur zugeführt werden, mit einem Grundkörper und einer Halte- und Spannvorrichtung für das axiale Spannen des Venenstückes. Ein solches Gerät ist aus US-A-4 232 659 bekannt.

Bei Gefässtransplantationen ist es unter Umständen erforderlich, ein aus einer Extremität (Bein oder Arm) herausoperiertes Venenstück vorübergehend ausserhalb des Körpers aufzubewahren. Dabei wird verhindert, dass das aus dem Körper herausgelöste Venenstück schrumpft und bei oder vor dem Wiedereinsetzen gestreckt und gedehnt werden muss, was zu starken Belastungen und Schädigungen der in dem Venenstück enthaltenden lebenden Zellen führt. Eine Aufgabe der Erfindung ist es, ein Gerät zu schaffen, das eine zellenschonende Lagerung herausoperierter Venenstükke ermöglicht.

Weiterhin ist es bekannt, dass bei alloplastischen Gefässprothesen für kleine Gefässe deren Innendurchmesser beispielsweise höchstens 6 mm ist - die Bildung von Thromben dauerhaft nur verhindert werden kann, wenn die alloplastische Prothese innen mit patienteneigenen (autologen) Epithel-, d.h. Endothelzellen, beschichtet werden. Ein dabei häufig angewandtes Verfahren sei im folgenden beschrieben:

In einer ersten Operation und unter Lokalanästhesie wird an geeigneter Stelle - z.B. am Unterarm - dem Patienten ein Stück oberflächlicher Vene entnommen, mit einer Länge, die den anatomischen Gegebenheiten angepasst ist und beispielsweise ungefähr 5 cm betragen kann. Das exzidierte Gefässtück muss dann aus dem Operationssaal in ein geeinetes biomedizinisches Labor gebracht werden. Dort werden die Endothelzellen von der Innenseite des Venenstückes abgelöst, in einer Zellwachstums-Kultur vermehrt und schliesslich auf die Innenseite der künstlichen Prothese aufgebracht. Dieses mit den patienteneigenen Endothelzellen ausgekleidete künstliche Gefäss wird in einer zweiten Operation, der Hauptoperation, dem Patienten eingesetzt.

Es liegt auf der Hand, dass dieser Vermehrungsprozess für die Zellen äusserst schwierig ist. Je grösser daher die Zahl der aus dem Venenstück gewonnenen lebenden Zellen zu Beginn ist, desto besser sind die Anfangsbedingungen für das Wachstum und desto kleiner ist die Anzahl notwendiger Vermehrungsschritte bis zur benötigten Endmenge an lebenden Zellen. Daher muss die Zellgewinnung unter optimalen Bedingungen durchgeführt werden. Es ist somit ein weiteres Ziel dieser Anmeldung, ein Gerät zu schaffen, mit dessen Hilfe aus einem Venenstück möglichst viele lebende und lebensfähige Endothelzellen gewonnen werden können.

Die beiden geschilderten Teilaufgaben werden mit einem Gerät der eingangs beschriebenen Art gelöst durch einen Grundkörper aus bioinertem Werkstoff, der eine von aussen zugängliche, jedoch durch ein Verschlusselement mit Hilfe von Dichtungen gasdicht verschliessbare Venenkammer enthält, ferner durch zwei Strömungskanäle, die vom zentralen Bereich der Venenkammer an die Aussenseite des Grundkörpers führen, weiterhin durch die Anordnung der Halte- und Spannvorrichtung zwischen den zentralen Strömungskanälen für das axiale Spannen des Venenstücks und schliesslich durch mindestens einen Strömungsweg zum Einbringen einer Flüssigkeitsfüllung in den peripheren Bereich und zum Entlüften, gegenbenenfalls Evakuieren, des peripheren Bereichs der Venenkammer.

Grundsätzlich werden heute zwei Verfahren angewandt, Endothelzellen aus einem Blutgefäss, in diesem Fall also aus einem Venenstück, zu gewinnen.

Bei dem einen Verfahren der Kanülierung wird beiderseits in das Venenstück eine Nadel geeigneten Durchmessers eingeführt und mit Hilfe von Ligaturen, d.h. durch Abbinden, auf den Kanülen fixiert. Das Venenstück wird zunächst mit physiologischer Kochsalzlösung gespült, um Blutreste zu entfernen. Dann wird eine definierte enzymatische Lösung von Kollagenase oder eines Kollagenase-Trypsin-Gemisches eingefüllt. Die beiden Kanülen werden daraufhin abgedichtet oder verschlossen und das Venenstück in eine wiederum mit Kochsalzlösung gefüllte Petrischale eingelegt; es verbleibt dort eine vorgegebene Zeit bei einer Temperatur, die ungefähr der Körpertemperatur von etwa 37° C entspricht. Um das Ablösen der Zellen zu begünstigen, kann das Venenstück mit den Fingern von aussen her zeitweise vorsichtig massiert werden. Ein Zellen-"Sediment" gewinnt man durch Zentrifugieren der aus dem Gefäss ausgespülten Lösung. Aus diesem Sediment erhält man schliesslich durch Resuspendieren in ein geeignetes Nährmedium der Wachstumskultur die zur Vermehrung bestimmten lebenden Endothelzellen.

Bei dem anderen Verfahren, dem Stülpen (Evertieren), wird das exzidierte Venenstück an einem Ende mit einer kleinen Länge auf einen Glasstab aufgeschoben und mit Hilfe einer Ligatur fixiert. Danach wird das ganze Venenstück gestülpt, so dass die Innenseite über den Glasstab nach aussen zu liegen kommt. Die Zellgewinnung selbst kann entweder durch Abkratzen - ohne Verwendung von Enzymen - mit Hilfe eines geigneten Miniaturschabers (Rubber-Policeman) erfolgen oder man kann die umgestülpte Vene in eine Enzymlösung tauchen - auch hier Kollagenase oder Kollagenase-Trypsin-Gemisch - und bei 37° C eine vorgegebene Zeit einlagern und dann mit dem gleichen Schaber abkratzen; eine weitere Möglichkeit ist die gleiche Methode anzuwenden, jedoch das mechanische Abkratzen durch ein Abspritzen mit Flüssigkeit zu ersetzen.

Ganz allgemein ist zu sagen, dass die Kanülierung ein schonenderes Verfahren ist als das Stülpen. Durch das Evertieren der Vene ist eine gewisse Schädigung der Zellen unvermeidbar. Wendet man das Abkratzen an, so muss damit gerechnet werden, dass die unter den Endothelzellen liegenden Gewebeschichten geschädigt werden. Dies führt dann zu äusserst unerwünschten Kontaminationen der Zellenkultur mit fremden Zelltypen, im vorliegenden Fall mit Fibroblasten oder glatten Muskelzellen.

In dem erfindungsgemässen Gerät wird daher die Methode des Kanülierens angewendet; es bietet zusätzlich zu den erwähnten Vorzügen dieses Verfahrens eine sehr einfache Handhabung - ein Vorteil, der bei der Arbeit unter sterilen Bedingungen nicht zu vernachlässigen ist - und eine reproduzierbare, sehr hohe Zellausbeute. So werden beispielsweise bis zu 90 % der im Venenstück enthaltenen Endothelzellen gewonnen.

Da nicht alle bei einer Exzision erhaltenen Venenstücke die gleiche Länge haben, ist es voreilhaft, wenn die Spannstrecke der Halte- und Spannvorrichtung in ihrer Länge veränderbar ist.

Die Strömungswege zum Einbringen einer Füllflüssigkeit in den peripheren Bereich der Venenkammer können zusätzlich einen evakuierbaren Pufferraum enthalten, um die Druckdifferenz zwischen beiden Seiten der "Wand" des Venenstückes variieren zu können.

Eine bevorzugte konstruktive Ausbildung des neuen Gerätes ergibt sich, wenn der Grundkörper ein Kreiszylinder ist, in dessen Stirnflächen die Strömungskanäle des zentralen Bereiches der Venenkammer sowie die Strömungswege für das Füllen und Entlüften bzw. Evakuieren des peripheren Bereiches der Venenkammer enden, und wenn weiterhin die Venenkammer durch die Mantelfläche des Kreiszylinders zugänglich ist; als Verschlusselement hat sich hierbei eine auf den Grundkörper axial aufschiebbare transparente Hülse bewährt. Schliesslich ist es von Vorteil, wenn der Grundkörper dabei zusätzlich an einer Stirnseite eine Schulter als Anschlag für die aufgeschobene Hülse aufweist.

Im folgenden wird die Erfindung anhand von Ausführungsbeispielen im Zusammenhang mit der Zeichnung näher erläutert.
- Fig. 1: ist ein Längsschnitt I-I von Fig. 2 durch eine Ausführungsform des neuen Grätes;
- Fig. 2: gibt den Schnitt II-II von Fig. 1 wieder;
- Fig. 3: schliesslich zeigt in gleicher Darstellung wie Fig. 1 eine weitere Ausführungsform des neuen Gerätes, während
- Fig. 4: eine Ansicht von Fig. 3 von links wiedergibt.

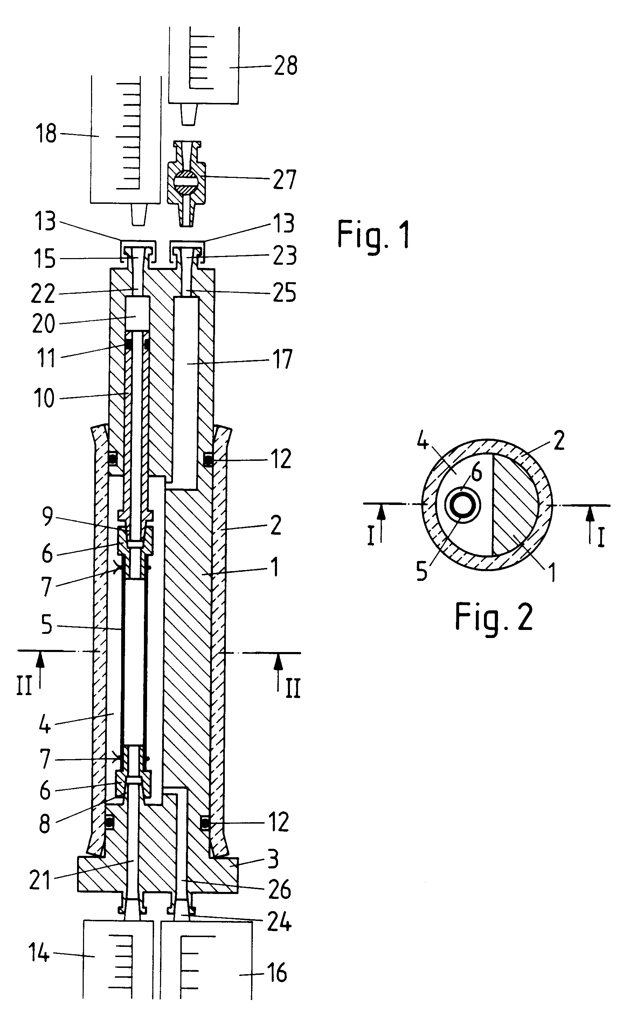

Das Gerät besteht aus einem Grundkörper 1 (Fig. 1), welcher aus einem biologisch inerten Werkstoff gefertigt ist. Auf diesem Grundkörper 1 ist eine transparente Hülse 2 geschoben, die als Verschlusselement für eine Venenkammer 4 dient. Der Grundkörper 1 weist als Anschlag für die transparente Hülse 2 und als Fixierung für eine nicht näher dargestellte Halterung an einem Ende eine Schulter 3 auf. In der aus dem Grundkörper 1 ausgesparten Venenkammer 4 ist ein Venenstück 5 unter leichter axialer Vorspannung gehalten.

Das Venenstück 5 ist - bei der Entnahme aus dem Patienten im Operationssaal - noch ausserhalb des Grundkörpers 1 auf zwei kurze mit einer zentralen Durchgangsbohrung versehene Anschlussteile 6 aufgezogen und mit Ligaturen 7, die im allgemeinen aus chirurgischem Nahtmaterial bestehen, befestigt. Die Anschlussteile 6 besitzen konische Innenflächen, die auf entsprechende Vaterkonen 8 und 9 aufgesteckt sind. Während der Konus 8 fest mit dem Grundkörper 1 verbunden bzw. ein Teil von ihm ist, ist der Konus 9 an einem Ende eines Rohres 10 das in einem Längskanal 20 verschiebbar ist. Dadurch kann man die freie Wegstrecke zwischen den Anschlussteilen 6 an die jeweilige Länge des Venenstückes 5 anpassen. Da das Venenstück 5 möglichst auf die ursprüngliche Länge im Körper vor der Entnahme gedehnt werden soll, wird es mit Hilfe des Rohres 10 gleichzeitig gespannt. Ein O-Ring 11 dient dabei als Dichtung und zugleich durch seine Wandreibung als Haltekraft für das unter Zug stehende Rohr 10.

In den zentralen Bereich der Venenkammer 4 bzw. in den Längskanal 20 münden zwei Strömungskanäle 21 und 22, die an gegenüberliegenden Stirnseiten des Grundkörpers 1 enden.

Aus dem peripheren Bereich der Venenkammer 4 führen zwei weitere Hohl- oder Strömungswege 25, 26 ebenfalls in die Stirnseiten des Grundkörpers 1. Der eine 25 dieser beiden Hohlwege ist mit einem Pufferraum 17 versehen. Die stirnseitigen Enden der Hohl- oder Strömungswege 25 und 26 sind identisch wie diejenigen der Strömungskanäle 21, 22 ausgebildet.

Die Enden der Strömungskanäle bzw. -wege 21, 22 bzw. 25, 26 sind als Mutterkonen 23 für entsprechende Gegenkonen 24 handelsüblicher Injektionsspritzen 14, 16, 18 ausgebidet. Die Oeffnungen 15 der Konen 23 sind durch Verschlusskappen 13 verschlossen, die beispielsweise als Drehverschlüsse ausgebildet sind.

Das "Einlegen" des auf den Anschlussteilen 6 befestigten Venenstückes 5 in die Venenkammer 4 erfolgt bei abgezogener Hülse 2. Für die weitere Handhabung des neuen Gerätes empfiehlt sich folgendes Vorgehen:

Nach dem beschriebenen Einlagern des Venenstückes 5 in die Kammer 4 wird zunächst die transparente Hülse 2 über die Kammer 4 geschoben, wobei zwei Dichtungsringe 12 einen gasdichten Abschluss bewirken und die Kammer 4 vor Kontaminationen schützen.

Nach dem Schliessen der Venenkammer 4 werden die Verschlusskappen 13 auf den zentralen Kanälen 21 und 22 geöffnet und an den Kanal 21 eine handelsübliche Injektionsspritze 14 angesetzt; das Venenstück 5 wird nun mit Hilfe der Spritze 14 kräftig von einer Spüllösung durchspült bis keine Blutreste mehr vorhanden sind. Die Spüllösung, die eine physiologische Kochsalzlösung ist, dient gleichzeitig auch als Konservierungsflüssigkeit, die im Innern des Venenstückes 5 verbleibt, wenn die Spülspritze 14 entfernt und die Oeffnungen 15 mit den beiden Verschlusskappen 13 wieder verschlossen sind. Anschliessend werden die Verschlusskappen 13 zur Freigabe der peripheren Strömungswege 25, 26 geöffnet und mittels der handelsüblichen Spritze 16, die an den Strömungsweg 26 angesetzt wird, werden die peripheren Bereiche der Venenkammer 4, beispielsweimse ebenfalls mit physiologischer Kochsalzlösung, gefüllt. Die "äussere" Kochsalzlösung verhindert das Austrocknen der äusseren Schichten des Venenstückes 5. Im Pufferraum 17 verbleibt jedoch ein Luftpolster, so dass dieser Raum als Druckausgleichsgefäss wirkt. Damit wird ein länger andauerndes Kollabieren des Venenstückes 5 verhindert; ein solches Kollabieren würde zu Schädigungen der zu gewinnenden lebenden Endothelzellen führen.

Soll das Venenstück 5 in radialer Richtung etwas stärker gedehnt werden, so kann im Raum 17 durch eine weitere Spritze 28, die über ein Absperrorgan 27 an das Ende des Strömungsweges 25 angesetzt wird, ein leichter Unterdruck erzeugt werden.

Nach dem Füllen der peripheren Venenkammer 4 mit Kochsalzlösung werden die Oeffnungen 15 der Hohlwege 25, 26 wieder mit den Kappen 13 verschlossen, wobei bei Unterdruck im Raum 17 eine Kappe 13 selbstverständlich auf das Absperrorgan 27 aufgesetzt wird. Das ganze Gerät wird nun in einer nicht dargestellten Verpackung, welche die Sterilität gewährt, zur Gewinnung der Endothelzellen in ein Labor transportiert.

Die Gewinnung der Endothelzellen erfolgt beispielsweise auf nachstehende Weise:
Die beiden Verschlusskappen 13 werden von den Kanälen 21, 22 entfernt und die Spül- bzw. Konservierungsflüssigkeit wird entleert. Falls dabei auch ein nur kurzzeitiges Kollabieren des Venenstückes 5 verhindert werden soll, ist es zweckmässig, im peripheren Bereich der Kammer 4 einen Unterdruck zu erzeugen bzw. zu verstärken. Ueber den Strömungskanal 21 wird nunmehr das Venenstück 5 mittels einer weiteren Spritze 14 mit einer Enzymlösung gefüllt und mit einer an den Strömungsweg 22 angeschlossenen weiteren Spritze 18 synchron hin- und herbewegt. Durch Aufbau eines geringen Ueberdruckes innerhalb des Venenstückes 5 wird dieses überdehnt, beispielsweise um 2 bis 3 % seines ursprünglichen Durchmessers, wobei aus der Kammer 4 verdrängte Flüssigkeit in den Pufferraum 17 überströmt. Experimentelle Versuche haben gezeigt, dass durch synchrones Bewegen der beiden Spritzen 14 und 18 die Zellausbeute erheblich gesteigert wird. Dieser Vorgang ersetzt das früher beschriebene Massieren des Venenstückes 5 von aussen und sichert, dass die von ihrer Unterlage gelösten Endothelzellen durch die auftretenden Strömungskräfte mitgerissen, jedoch nicht geschädigt werden. Nach Entfernen der Spritze 18 wird schliesslich die Enzymlösung mit den Endothelzellen in die Spritze 14 abgesaugt. Die so gewonnenen Endothelzellen sind dank dieses schonenden Verfahrens praktisch frei von fremden Zellen, wie Fibroblasten oder glatten Muskelzellen.

Das zweite Ausführungsbeispiel nach 3 und 4 ist konstruktiv etwas einfacher gehalten als die Ausführungsform nach Fig. 1 und 2; da die einzelnen Einzelelemente weitgehend mit denjenigen von Fig. 1 und 2 übereinstimmen, sind soweit wie möglich die gleichen Bezugsziffern verwendet.

Der Grundkörper 1 ist hier ein rechteckiges flaches Gefäss aus bioinertem Werkstoff, an dem drei mit den Verschlusskappen 13 verschlossene Oeffnungen 15 für den Anschluss konventioneller Spritzen 14, 16 und 18 vorgesehen sind. Als Verschlusselement für die Venenkammer 4 ist ein aufklappbarer durchsichtiger Deckel 30 vorhanden, der über ein Scharnier 31 an einer Flachseite des Grundkörpers 1 drehbar befestigt ist. Für die Abdichtung der Venenkammer 4 nach aussen dient wiederum eine Dichtung 12, die als geschlossener Ring die im Grundriss stadionartig geformte Venenkammer 4 umgibt und von dem geschlossenen Deckel 30 zusammengepresst wird.

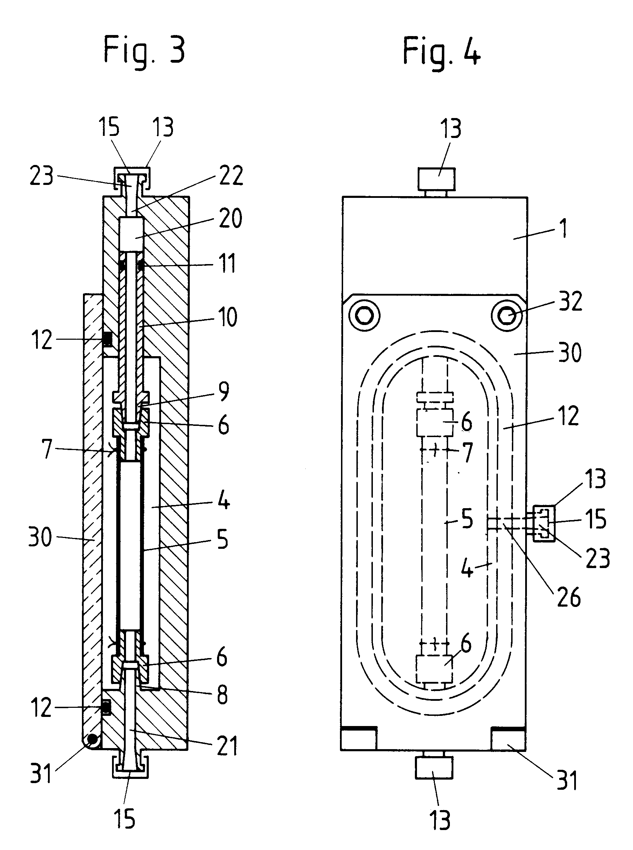

Als Abschluss der Venenkammer 4 wird der Deckel 30 gehalten durch zwei Schrauben 32, die in Fig. 4 schematisch angedeutet sind und in den Grundkörper 1 eingeschraubt werden.

Die Einfüllöffnung 26 für das Einfüllen eine physiologischen Kochsalzlösung in den Aussenraum der Venenkammer 4 ist bei diesem Beispiel seitlich angeordnet und mündet in eine Längsseite der Venenkammer 4. Ein Pufferraum, der dem Raum 17 des ersten Beispiels entspricht, wird bei dieser Konstruktion dadurch erzeugt, dass der Aussenraum der Venenkammer 4 nur unvollständig mit Flüssigkeit gefüllt wird.

Der übrige Aufbau und die Verwendung des Gerätes entsprechen bei der zweiten Ausführungsform dem im Zusammenhang mit Fig. 1 und 2 Gesagten.

Da alle im menschlichen oder tierischen Körper vorhandenen Hohlgefässe auf ihren Innenseiten mit einer besonderen Schicht Epithelzellen ausgekleidet sind, ist selbstverständlich das Gerät auch für die Gewinnung dieser anderen Epithelzellen verwendbar.

## Patentansprüche

1. Gerät zur Lagerung und/oder Behandlung operativ gewonnener Venenstücke (5), insbesondere zum Transport und/oder zur Gewinnung von Endothelzellen, die enzymatisch aus dem Venenstück herausgelöst und zur Vermehrung einer geeigneten Zellwachstumskultur zugeführt werden, mit einem Grundkörper (1) und einer Halte- und Spannvorrichtung (6 bis 11) für das axiale Spannen des Venenstücks, dadurch gekennzeichnet, daß der Grundkörper (1) aus bioinertem Werkstoff besteht und eine von aussen zugängliche, jedoch durch ein Verschlusselement (2, 30) mit Hilfe von Dichtungen (12) gasdicht verschliessbare Venenkammer (4) enthält, daß ferner zwei Strömungskanäle (21, 22) vorgesehen sind, die vom zentralen Bereich der Venenkammer (4) an die Aussenseite des Grundkörpers (1) führen, daß weiterhin die Halte- und Spannvorrichtung (6 bis 11) zwischen den zentralen Strömungskanälen (21, 22) für das axiale Spannen des Venenstückes (5) angeordnet ist, und schliesslich, daß mindestens ein Strömungsweg (25, 26) zum Einbringen einer Flüssigkeitsfüllung in den peripheren Bereich und zum Entgasen des peripheren Bereichs der Venenkammer (4), vorgesehen ist.

2. Gerät nach Anspruch 1, dadurch gekennzeichnet, dass die Spannstrecke der Halte- und Spannvorrichtung (6 bis 11) in ihrer Länge veränderbar ist.

3. Gerät nach Anspruch 1 oder 2, dadurch gekennzeichnet, dass ein Strömungsweg (25 bzw. 26) zum Einbringen der Flüssigkeitsfüllung einen evakuierbaren Pufferraum (17) enthält.

4. Gerät nach einem der Ansprüche 1 bis 3, dadurch gekennzeichnet, dass der Grundkörper (1) ein Kreiszylinder ist, in dessen Stirnflächen die Strömungskanäle (21, 22) des zentralen Bereiches der Venenkammer (4) sowie die Strömungswege (25, 26) für das Füllen und Entlüften/Evakuieren des peripheren Bereiches der Venenkammer (4) enden, und dass weiterhin die Venenkammer (4) durch die Mantelfläche des Kreiszylinders zugänglich ist.

5. Gerät nach Anspruch 4, dadurch gekennzeichnet, dass der Grundkörper (1) an einer Stirnseite eine Schulter (3) trägt.

6. Gerät nach Anspruch 4, dadurch gekennzeichnet, dass das Verschlusselement (2) eine auf den Grundkörper (1) axial aufschiebbare Hülse ist.

## Claims

1. A device for storing and/or treating surgically obtained pieces of veins (5), more particularly for conveying and/or obtaining endothelial cells which are separated from the piece of vein by enzymatic action and are supplied to a suitable cell growth culture for propagation, the device comprising a main member (1) and a device (6 to 11) for holding and axially clamping the piece of vein, characterised in that the main member (1) is made of bioinert material and contains a vein chamber (4) which is externally accessible but closable in gas-tight manner by a closure component (2, 30) in co-operation with seals (12), two flow ducts (21, 22) are provided and lead from the central region of the vein chamber (4) to the outside of the main member (1), the holding and clamping device (6 to 11) is disposed between the central flow ducts (21, 22) for axially clamping the piece of vein (5), and at least one flow passage (25, 26) is provided for introducing a liquid into and degassing the peripheral region of the vein chamber.

2. A device according to claim 1, characterised in that the clamping section of the holding and clamping device (6 to 11) has a variable length.

3. A device according to claim 1 or 2, characterised in that a flow passage (25 or 26) for introducing the liquid contains a buffer chamber (17) which can be evacuated.

4. A device according to any of claims 1 to 3, characterised in that the main member (1) is a circular cylinder having end surfaces into which the flow channels (21, 22) for the central region of the vein chamber (4) and the flow passages (25, 26) for filling and venting or evacuating the peripheral region of the vein chamber (4) end, and the vein chamber (4) is accessible through the generated surface of the circular cylinder.

5. A device according to claim 4, characterised in that the main member (1) has a shoulder (3) at one end.

6. A device according to claim 4, characterised in that the closure member (2) is a sleeve which can be axially slid over the main member (1).

## Revendications

1. Appareil d'entreposage et/ou de traitement de morceaux de veine (5) prélevés par opération, en particulier pour le transport et/ou la récupération de cellules endothéliales qui sont détachées du morceau de veine par voie enzymatique et placées dans une culture convenable de croissance de cellules pour leur multiplication, appareil comprenant un corps de base (1) et un dispositif de fixation et de tension (6 à 11) destiné à la mise sous tension axiale du morceau de veine, caractérisé en ce que le corps de base (1) est en matière bioinerte et renferme une chambre à veine (4) accessible de l'extérieur, mais obturable de façon étanche aux gaz par un élément de fermeture (2, 30) et à l'aide de joints (12), en ce que par ailleurs deux canaux de circulation (21, 22) sont prévus et mènent de la partie centrale de la chambre à veine (4) au côté extérieur du corps de base (1), en ce que de plus le dispositif de fixation et de traction (6 à 11) est disposé entre les canaux centraux de circulation (21, 22) pour la mise sous traction axiale du morceau de veine (5) et finalement en ce qu'au moins une voie de circulation (25, 26) est prévue pour l'introduction d'un liquide de remplissage de la région périphérique et pour la purge de la région périphérique de la chambre à veine (4).

2. Appareil selon la revendication 1, caractérisé en ce que l'intervalle d'étirage du dispositif de fixation et de traction (6 à 11) a une longueur qui est modifiable.

3. Appareil selon la revendication 1 ou 2, caractérisé en ce que la voie de circulation (25 ou 26) destinée à l'introduction du liquide de remplissage comprend une chambre tampon (17) pouvant être mise sous vide.

4. Appareil selon l'une des revendications 1 à 3, caractérisé en ce que le corps de base (1) est un cylindre à base circulaire dans les surfaces extrêmes duquel débouchent les canaux de circulation (21, 22) de la partie centrale de la chambre à veine (4) ainsi que les voies de circulation (25, 26) destinées au remplissage et à la purge/la mise sous vide de la région périphérique de la chambre à veine (4), et en ce que par ailleurs la chambre à veine (4) est accessible par la surface de l'enveloppe du cylindre à base circulaire.

5. Appareil selon la revendication 4, caractérisé en ce que le corps de base (1) comporte un épaulement (3) sur un côté extrême.

6. Appareil selon la revendication 4, caractérisé en ce que l'élément obturateur (2) est un manchon pouvant s'enfiler axialement sur le corps de base (1).
